# NEUE EUROPÄISCHE PATENTSCHRIFT

(11) **EP 0 414 929 B2**
(45) Veröffentlichungstag und Bekanntmachung des Hinweises auf die Entscheidung über den Einspruch: **22.12.1999**
(45) Hinweis auf die Patenterteilung: 15.03.1995
(21) Anmeldenummer: 89115851.1
(22) Anmeldetag: 28.08.1989
(51) Int. Cl.: A61N 1/36, H01R 29/00

(54) **Wahlweise bipolar oder unipolar betreibbares medizinisches Gerät mit einem Anschluss für eine elektrische Signale zwischen dem Gerät und dem Körper eines Lebewesens leitende Elektrode**
Medical apparatus operable in either a bipolar or a unipolar configuration, having a connector for an electrode conducting a signal between the apparatus and a living being
Appareil médical utilisable au choix en mode bipolaire ou unipolaire, ayant un connecteur pour une électrode conductrice d'un signal électrique entre l'appareil et le corps d'un être vivant

(43) Veröffentlichungstag der Anmeldung: 06.03.1991
(73) Patentinhaber: Pacesetter AB, 175 84 Järfälla (SE)
(72) Erfinder: Engström, Jan, Dipl.-Ing., S-163 55 Spanga (SE)
(74) Vertreter: Harrison, Michael Charles

(56) Entgegenhaltungen:
- US-A- 4 236 525
- US-A- 4 301 805
- US-A- 4 420 216
- US-A- 4 532 931
- US-A- 4 592 360

## Beschreibung

Die Erfindung betrifft ein medizinisches Gerät nach dem Oberbegriff des Anspruchs 1, an das wahlweise eine bipolare oder eine unipolare Elektrode zum Leiten elektrischer Signale zwischen dem Gerät und dem Körper eines Lebewesens anschliessbar ist und das einen ein Signalpotential und einen ein Bezugspotential führenden Anschluss aufweist, wobei das Gerät wahlweise auf bipolaren oder unipolaren Betrieb umschaltbar ist und wobei im Falle des unipolaren Betriebs der das Bezugspotential führende Anschluss mit Mitteln zum Anlegen des Bezugspotentials an den Körper des Lebewesens leitend verbunden ist.

Dabei wird im Falle der Verwendung einer unipolaren Elektrode so vorgegangen, dass ein zur Zufuhr bzw. Ableitung der jeweiligen elektrischen Signale geeigneter Bereich des Körpers des Lebewesens mittels der unipolaren Elektrode mit dem das Signalpotential führenden Anschluss verbunden wird, während das Bezugspotential an eine andere Stelle des Körpers des Lebewesens mit Hilfe der Mittel zum Anlegen des Bezugspotentials angelegt wird. Eine unipolare Elektrode weist daher in der Regel nur einen Leiter auf. Im Falle der Verwendung einer bipolaren Elektrode werden sowohl der das Signal- als auch der das Bezugspotential führende Anschluss mit einem zur Zufuhr bzw. Ableitung der jeweiligen elektrischen Signale und zum Anlegen des Bezugspotentials geeigneten Bereich des Körpers mittels der bipolaren Elektrode verbunden. Eine bipolare Elektrode besitzt daher zwei Leitungen, die in relativ dicht beieinander, liegenden, für Kontaktierung des im wesentlichen gleichen Bereichs des Körpers des Lebewesens vorgesehenen Kontaktteilen enden. Es wird somit deutlich, dass es zwar möglich ist, mit einer bipolaren Elektrode die elektrischen Signale auch unipolar zu leiten, indem nur ein Leiter der bipolaren Elektrode mit einem Anschluss des Geräts, und zwar dem das Signalpotential führenden Anschluss, verbunden wird, während das Bezugspotential auf andere Weise an den Körper des Lebewesens angelegt wird. Umgekehrt ist es jedoch offensichtlich unmöglich, mittels einer unipolaren Elektrode bipolar elektrische Signale zu leiten.

Es ist ein Gerät der eingangs genannten Art bekannt, das als Herzschrittmacher ausgebildet ist. Dieses Gerät weist also Mittel zum Stimulieren der Herztätigkeit eines Lebewesens und Mittel zum Detektieren von Herzschlägen auf, wobei von den Mitteln zum Stimulieren Stimulationsimpulse mittels einer Elektrode zu dem Herz des Lebewesens geleitet werden, während der elektrischen Aktivität des Herzens entsprechende Signale über eine Elektrode zu den Mitteln zum Detektieren geleitet werden. Es handelt sich bei dem bekannten Herzschrittmacher um ein in den Körper des Lebewesens implantierbares Gerät, an das wahlweise eine unipolare oder eine bipolare endokardiale Elektrode anschliessbar ist, die durch das Venensystem des Patienten in das entsprechende Atrium oder Ventrikel des Herzens geführt und dort verankert ist. Da es auch nach der Implantation des Gerätes notwendig sein kann, von bipolarem auf unipolaren Betrieb und umgekehrt überzugehen, kann die entsprechende Umschaltung mit Hilfe eines externen Geräts, eines sogenannten Programmers, telemetrisch erfolgen. Es versteht sich, dass als unabdingbare Voraussetzung für den Übergang von dem unipolaren zu dem bipolaren Betrieb eine bipolare Elektrode mit dem Herzschrittmacher verbunden sein muss.

Ein Übergang von bipolarer auf unipolare Stimulation kann z.B. zweckmässig sein, weil bei den meisten Patienten die zur Erzielung einer Stimulation benötigte Energiemenge bei unipolarer Stimulation geringer ist. Ein Übergang von unipolarer auf bipolare Stimulation ist dann angezeigt, wenn in demjenigen Bereich, in dem das Bezugspotential an den Körper des Lebewesens angelegt wird, im Falle eines Herzschrittmachers also normalerweise im Bereich von dessen elektrisch leitendem und mit dem das Bezugspotential führenden Anschluss des Gerätes elektrisch leitend verbundenem Gehäuse, unerwünschte Stimulationseffekte, z.B. Muskelzuckungen, auftreten. Ein Übergang von unipolarer auf bipolare Detektion kann sinnvoll sein, wenn bei unipolarer Detektion starke Störungen (noise) auftreten. Derartige Störungen treten bei bipolarer Detektion meist weniger stark auf. Während der Übergang von bipolarem auf unipolaren Betrieb unkritisch ist, macht es die Umschaltung von unipolarer auf bipolare Betriebsweise insbesondere nach bereits erfolgter Implantation erforderlich, dass sich der behandelnde Arzt sorgfältigst davon überzeugt, dass tatsächlich eine bipolare Elektrode mit dem Herzschrittmacher verbunden ist. Im Falle eines Irrtums ist eine ordnungsgemässe Stimulation nicht mehr gewährleistet, so dass bei vollständig oder in hohem Masse auf die Stimulation durch den Herzschrittmacher angewiesenen Patienten bedrohliche Situationen auftreten können.

Der Erfindung liegt daher die Aufgabe zugrunde, ein Gerät der eingangs genannten Art so auszubilden, dass Irrtümer über die Art der angeschlossenen Elektrode bei der Umschaltung von unipolarem auf bipolaren Betrieb für den Patienten ohne Folgen bleiben.

Es ist durch US-A-4 532 931 schon ein Gerät bekannt, das als Herzschrittmacher mit unipolarer Stimulation und automatischer Umschaltung zwischen unipolarer und bipolarer Detektion ausgebildet ist. Die automatische (d.h ohne Zutun des Arztes) Umschaltung zwischen den Detektionsarten erfolgt dadurch, dass eine erste und eine zweite Impedanz, je wesentlich grösser als die Impedanz des Körpergewebes und wesentlich niedriger als die Impedanz des Eingangsverstärkers, wobei die erste und die zweite Impedanz zwischen dem Herzschrittmacherkapsel und dem Bezugspotentialanschluss für die Ringelektrode beziehungsweise zwischen dem Herzschrittmachergehäuse und dem Signalpotentialanschluss für die Spitzenelektrode verbunden sind, und welche in Abhängigkeit davon, ob eine bipolare oder eine unipolare Elektrode an dem Gerät angeschlossen ist, das Signal, das den Verstärker erreicht bestimmen. Wenn bei diesem Gerät keine bipolare Elektrode angeschlossen ist, liegt durch die zweite Impedanz eine elektrisch leitende Verbindung zwischen dem Bezugspotentialanschluss und dem Gehäuse vor. Diese leitende Verbindung liegt deshalb auch bei unipolarer Detektion vor, doch ohne dass die Verbindung dabei erst hergestellt wird.

Nach der Erfindung, wie sie im unabhängigen Anspruch definiert ist, wird diese Aufgabe dadurch gelöst, dass erst bei Anschluss einer unipolaren Elektrode an das Gerät Zwangsläufig eine leitende Verbindung des das Bezugspotential führenden Anschlusses mit den Mitteln zum Anlegen des Bezugspotentials hergestellt wird. Durch die erfindungsgemässe Massnahme ist sichergestellt, dass auch dann, wenn eine unipolare Elektrode an das Gerät angeschlossen ist und in der irrtümlichen Annahme, es handele sich um eine bipolare Elektrode, auf bipolaren Betrieb umgeschaltet wird, eine leitende Verbindung zwischen dem das Bezugspotential führenden Anschluss und den Mitteln zum Anlegen des Bezugspotentials vorliegt. Damit sind Fehlfunktionen und eventuelle nachteilige Folgen für den Patienten mit Sicherheit vermieden.

Die Erfindung sieht vor, dass das Gerät elektrische Verbindungsmittel zum wahlweisen Anschluss der bipolaren oder der unipolaren Elektrode aufweist und dass die elektrischen Verbindungsmittel derart ausgebildet sind, dass erst bei Anschluss der unipolaren Elektrode zwangsläufig die leitende Verbindung des das Bezugspotential führenden Anschlusses mit den Mitteln zum Anlegen des Bezugspotentials hergestellt wird. Es werden also zum Anschluss einer Elektrode ohnehin erforderlich elektrische Verbindungsmittel derart ausgebildet, dass sich beim Anschluss einer unipolaren Elektrode die gewünschte leitende Verbindung zwangsläufig ergibt, so dass der erforderliche technische Aufwand äusserst gering ist.

Die elektrischen Verbindungsmittel können bevorzugt als Steckverbindung ausgeführt sein. Demnach wird also eine einen bequemen und einfachen Anschluss der jeweiligen Elektrode ermöglichende Steckverbindung so ausgebildet, dass sich beim Anschluss einer unipolaren Elektrode zwangsläufig die gewünschte leitende Verbindung zwischen dem das Bezugspotential führenden Anschluss des Gerätes und den Mitteln zum Anlegen des Bezugspotentials ergibt. Dabei kann gemäss einer Variante der Erfindung vorgesehen sein, dass die Mittel zum Herstellen einer leitenden Verbindung durch einen an dem der unipolaren Elektrode zugeordneten zweiten Verbindungsteil angebrachten Kontaktabschnitt gebildet sein können. Die Steckverbindung kann dann gemäss einer weiteren Variante der Erfindung in technisch einfacher und kostengünstiger Weise als koaxiale rotationssymmetrische Steckverbindung ausgeführt sein, wobei die Pole des dem Gerät zugeordneten ersten Verbindungsteils axial aufeinanderfolgend angeordnet sind und der zweite und der dritte Pol einander benachbart sind.

Die Erfindung besitzt dann besondere Vorteile, wenn gemäss einer Variante der Erfindung das Gerät in den Körper des Lebewesens implantierbar ist und telemetrisch auf unipolaren oder bipolaren Betrieb umschaltbar ist. In diesem Falle ist es nach der Implantation des Geräts besonders schwierig zu erkennen, ob tatsächlich eine bipolare Elektrode angeschlossen ist, so dass eine irrtümliche Umschaltung auf bipolaren Betrieb besonders leicht erfolgen kann und die Abwendung negativer Folgen für den Patienten demzufolge besonders wichtig ist.

Die Erfindung ist nachfolgend anhand eines in den Figuren dargestellten Ausführungsbeispiels erläutert. Es zeigen:
- FIG 1: einen erfindungsgemäss ausgebildeten Herzschrittmacher, an den wahlweise unipolare oder bipolare Elektroden anschliessbar sind, in teilweise schematische Darstellung,
- FIG 2: eine zum Anschluss an den erfindungsgemässen Herzschrittmacher bestimmte bipolare Elektrode in teilweise geschnittener Darstellung, und
- FIG 3: eine zum Anschluss an den erfindungsgemässen Herzschrittmacher bestimmte unipolare Elektrode in teilweise geschnittener Darstellung.

Der erfindungsgemässe Herzschrittmacher, der zur Implantation in den Körper eines Lebewesens vorgesehen ist, besitzt einen Stimulationsimpuls-Generator (1) und eine Detektorschaltung (2), die jeweils mit einer Steuerlogik (3) verbunden sind. Die Steuerlogik (3) veranlasst den Stimulationsimpuls-Generator (1) immer dann zur Abgabe eines elektrischen Stimulationsimpulses, wenn im Anschluss an einen mittels der Detektorschaltung (2) detektierten natürlichen Herzschlag oder einen von dem Stimulationsimpuls-Generator (1) abgegebenen Stimulationsimpuls nach Ablauf eines einer bestimmten Herzschlagfrequenz entsprechenden Zeitintervalls mittels der Detektorschaltung (2) kein natürlicher Herzschlag detektiert wurde. Der Stimulationsimpuls-Generator (1) besitzt einen das Stimulationspotential führenden Ausgang (4a) und einen ein Bezugspotential führenden Ausgangsanschluss (4b). In entsprechender Weise weist die Detektorschaltung (2) einen Eingang (5a), an dem das der elektrischen Aktivität des zu stimulierenden Herzens entsprechende Potential liegt, und einen Eingangsanschluss (5b) auf, der das Bezugspotential führt.

Da die Leitung der Stimulationsimpulse von dem Stimulationsimpuls-Generator (1) zu dem zu stimulierenden Herz und die Leitung der der elektrischen Aktivität des Herzens entsprechenden Signale zu der Detektorschaltung (2) über eine gemeinsame Elektrode erfolgen soll, ist zum einen der Ausgang (4a) des Stimulationsimpuls-Generators (1) mit dem Eingang (5a) der Detektorschaltung (2) und zum anderen der Ausgangsanschluss (4b) des Stimulationsimpuls-Generators (1) mit dem Eingangsanschluss (5b) der Detektorschaltung (2) verbunden.

Der Herzschrittmacher besitzt also einen das Signalpotential führenden Anschluss (6), der durch den Verbindungspunkt des Ausgangs (4a) des Stimulationsimpuls-Generators (1) mit dem Eingang (5a) der Detektorschaltung (2) gebildet ist, und einen das Bezugspotential führenden Anschluss (7), der durch den Verbindungspunkt des Ausgangsanschlusses (4b) des Stimulationsimpuls-Generators (1) mit dem Eingangsanschluss (5b) der Detektorschaltung (2) gebildet ist.

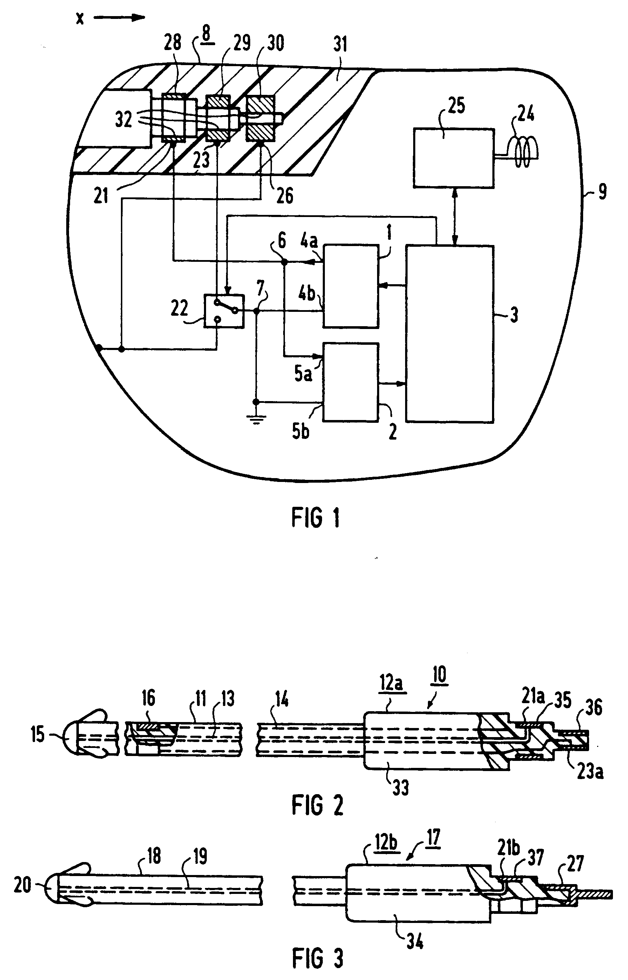

Eine in FIG 1 nicht dargestellte Elektrode muss also jedenfalls eine leitende Verbindung zwischen dem das Signalpotential führenden Anschluss (6) des Herzschrittmachers und dem Herz des Lebewesens herstellen. Der Anschluss (6) ist daher mit dem ersten Pol (21) einer zum Anschluss sowohl bipolarer als auch unipolarer Elektroden dienenden Steckverbindung verbunden, von der in FIG 1 nur das dem Herzschrittmacher zugeordnete erste Verbindungsteil (8) dargestellt ist.

Im Falle der Verwendung einer bipolaren Elektrode wird das Bezugspotential über die bipolare Elektrode direkt an das Herz angelegt. Das erste Verbindungsteil (8) besitzt daher einen zweiten Pol (23), der mittels eines als elektronischer Schalter ausgebildeten Umschalters (22) an den das Bezugspotential führenden Anschluss (7) des Herzschrittmachers anschaltbar ist. Die entsprechende Schaltstellung des Umschalters (22) ist in FIG 1 dargestellt. Im Falle der Verwendung einer unipolaren Elektrode wird das Bezugspotential über ein die elektronischen Komponenten des Herzschrittmachers umgebendes, in FIG 1 schematisch angedeutetes, hermetisch dichtes Gehäuse (9), das aus einem elektrisch leitenden Werkstoff, z.B. Titan, gebildet ist, an den Körper des Lebewesens angelegt. Dies geschieht, indem der Umschalter (22) in seine in FIG 1 nicht dargestellte Schaltposition gebracht wird, in der eine leitende Verbindung zwischen dem das Bezugspotential führenden Anschluss (7) des Herzschrittmachers und dem Gehäuse (9) besteht.

Das dem Herzschrittmacher zugeordnete erste Verbindungsteil (8) der Steckverbindung ist übrigens an dem Gehäuse (9) befestigt.

In FIG 2 ist eine bipolare Elektrode (10) dargestellt, die aus einer zweipoligen Leitung (11) und einem zweiten Verbindungsteil (12a) der Steckverbindung gebildet ist, wobei das zweite Verbindungsteil (12a) in das an dem Herzschrittmacher angebrachte erste Verbindungsteil (8) einführbar ist. Die Leitung (11) weist einen Mittelleiter (13) und einen beispielsweise als Metallgeflecht ausgeführten äusseren Leiter (14) auf, der den Mittelleiter (13) umgibt und von diesem elektrisch isoliert ist. Auch der äussere Leiter (14) ist isoliert. Der Mittelleiter (13) ist elektrisch leitend mit einer Spitze (15) verbunden, die mit dem Gewebe des zu stimulierenden Herzens in Kontakt gebracht wird. Der Mittelleiter (13) dient dazu, dem Herzen Stimulationsimpulse bzw. dem Herzschrittmacher der elektrischen Aktivität des Herzens entsprechende Signale zuzuführen. Der äussere Leiter (14) ist elektrisch leitend mit einem Ring (16) verbunden, der ebenfalls mit dem Gewebe des Herzens in Kontakt gebracht wird und dazu dient, das Herz auf das Bezugspotential zu legen.

Die in FIG 3 dargestellte unipolare Elektrode (17) ist entsprechend aufgebaut, allerdings fehlen hier der äussere Leiter (14) und der Ring (16). Die unipolare Elektrode (17) besitzt also eine einpolige Leitung (18) mit einem Leiter (19), der in der Spitze (20) endet und wie der Mittelleiter (13) der bipolaren Elektrode (10) zur Leitung der Stimulationsimpulse und der der elektrischen Aktivität des Herzens entsprechenden Signale dient, und ein zweites Verbindungsteil (12b). Wie bereits erwähnt wurde, wird im Falle der Verwendung der unipolaren Elektrode (17) das Bezugspotential nicht unmittelbar an das zu stimulierende Herz, sondern über das elektrisch leitende Gehäuse (9) (siehe FIG 1) des Herzschrittmachers an den Körper des Lebewesens angelegt.

Wie aus den Figuren 2 und 3 ersichtlich ist, besitzen die zweiten Verbindungsteile (12a und 12b) jeweils einen dem ersten Pol (21) des ersten Verbindungsteiles (8) entsprechenden ersten Pol (21a bzw. 21b), über den bei angeschlossener Elektrode (10 bzw. 17) der Mittelleiter (13) bzw. den Leiter (19) mit dem das Signalpotential führenden Anschluss (6) des Herzschrittmachers verbunden ist. Das der bipolaren Elektrode (10) zugeordnete zweite Verbindungsteil (12a) besitzt ausserdem einen dem zweiten Pol (23) des ersten Verbindungsteiles (8) entsprechenden zweiten Pol (23a), mit dem der Ring (16) über den äusseren Leiter (14) elektrisch leitend verbunden ist.

Ist also die bipolare Elektrode (10) an den Herzschrittmacher angeschlossen, kann in Abhängigkeit von der Schaltstellung des Umschalter (22) wahlweise bipolar oder unipolar stimuliert und detektiert werden. Dabei entspricht die in FIG 1 dargestellte Schaltstellung des Umschalters (22), wie sich aus den vorstehenden Erläuterung ergibt, der bipolaren Betriebsart, während die nicht gezeigte Schaltstellung der unipolaren Betriebsart entspricht. Die nicht gezeigte Schaltstellung muss der Umschalter (22) an sich auch dann einnehmen, wenn die unipolare Elektrode (17) an den Herzschrittmacher angeschlossen ist, da andernfalls infolge des Fehlens einer Verbindung zwischen dem das Bezugspotential führenden Anschluss (7) und dem Körper des den Herzschrittmacher tragenden Lebewesens Fehlfunktionen auftreten würden.

Der in FIG 1 dargestellte Herzschrittmacher besitzt einen mit einer Sende/Empfangs-Spule (24) verbundenen Telemetrieschaltkreis (25), der an die Steuerlogik (3) angeschlossen ist. Mittels eines nicht dargestellten externen Gerätes, das ebenfalls eine Sende/Empfangs-Spule aufweist, besteht also die Möglichkeit, auch nach der Implantation des Herzschrittmachers einen Datenaustausch zwischen dem Herzschrittmacher und dem externen Gerät vorzunehmen, sofern das externe Gerät relativ zu dem Körper des den Herzschrittmacher tragenden Lebewesens in eine solche Position gebracht wird, das eine induktive Kopplung der Sende/Empfangs-Spule des externen Gerätes mit der Sende/Empfangs-Spule (24) des Herzschrittmachers vorliegt. Dabei besteht u.a. die Möglichkeit, den Umschalter (22) telemetrisch zu betätigen, was durch eine zwischen dem Umschalter (22) und der Steuerlogik (3) vorgesehene Steuerleitung angedeutet ist, um den Herzschrittmacher von bipolarem auf unipolaren Betrieb und umgekehrt umschalten zu können.

Dies ist dann kein Problem, wenn eine bipolare Elektrode (10) angeschlossen ist, da diese ohne weiteres auch zur unipolaren Stimulation und Detektion verwendbar ist. Ist jedoch eine unipolare Elektrode (17) angeschlossen und wird der Herzschrittmacher irrtümlicherweise auf bipolaren Betrieb umgeschaltet, treten, wie bereits erläutert wurde, Fehlfunktionen auf.

Um diese Fehlfunktionen bei einer unipolaren Elektrode (17) zu verhindern, weist das erste Verbindungsteil (8) einen dritten Pol (26) auf, der ständig elektrisch leitend mit dem Gehäuse (9) des Herzschrittmachers verbunden ist, während das zweite Verbindungsteil (12b) der unipolaren Elektrode (17) einen Kontaktabschnitt (27) besitzt, der derart ausgebildet ist, dass er bei angeschlossener unipolarer Elektrode (17) eine elektrisch leitende Verbindung zwischen dem dritten Pol (26) und dem zweiten Pol (23) des ersten Verbindungsteiles (8) herstellt. Damit besteht bei angeschlossener unipolarer Elektrode (17) unabhängig von der Schaltstellung des Umschalters (22) eine elektrisch leitende Verbindung zwischen dem das Bezugspotential führenden Anschluss (7) des Herzschrittmachers einerseits und dem Gehäuse (9) des Herzschrittmachers andererseits. Es können somit keine Fehlfunktionen auftreten, wenn der Herzschrittmacher bei angeschlossener unipolare Elektrode (17) versehentlich auf bipolaren Betrieb umgeschaltet wird.

Wie aus den Figuren 1 bis 3 ersichtlich ist, ist die Steckverbindung als koaxiale rotationssymmetrische Steckverbindung ausgeführt. Die Pole des ersten Verbindungsteiles (8) sind durch drei axial im Abstand von einander angeordnete ringförmige Kontaktteile (28, 29, 30) gebildet. Diese sind in ein Trägerteil (31) aus einem elektrisch isolierenden Material eingebettet, das eine Bohrung zur Aufnahme des der bipolaren Elektrode (10) bzw. der unipolaren Elektrode (17) zugehörigen zweiten Verbindungsteiles (12a bzw. 12b) aufweist. Dabei bilden Bohrungswandungen der Kontaktteile (28, 29, 30), die deren Kontaktflächen darstellen und demzufolge freiliegen, Wandabschnitte der Bohrung (32). Die Bohrungsdurchmesser der Kontaktteile (28, 29, 30) nehmen in der durch einen Pfeil x angedeuteten Steckrichtung ab. Das Kontaktteil (28) bildet den ersten Pol, das Kontaktteil (29) den zweiten Pol und das Kontaktteil (30) den dritten Pol des ersten Verbindungsteiles (8).

Die zweiten Verbindungsteile (12a und 12b) weisen jeweils einen aus einem elektrisch isolierenden Werkstoff gebildeten Grundkörper (33 bzw. 34) auf. Die Grundkörper (33, 34) sind derart geformt, dass sie in die Bohrung (32) des ersten Verbindungsteiles (8) einführbar sind. Im Falle des zu der bipolaren Elektrode (10) gehörigen Verbindungsteiles (12a) sind dessen beide Pole durch zwei ringförmige Kontaktteile (35 und 36) gebildet, die derart angeordnet und bemessen sind, dass sie mit ihren zylindrischen Mantelflächen, die die Kontaktflächen bilden und demzufolge freiliegen, die Kontaktflächen der Kontaktteile (28 und 29) des ersten Verbindungsteiles kontaktieren, wenn das zweite Verbindungsteil (12a) in das erste Verbindungsteil (8) eingesteckt ist.

Der einzige Pol des der unipolaren Elektrode (17) zugeordneten Verbindungsteils (12b) ist durch ein Kontaktteil (37) gebildet, das hinsichtlich seiner Abmessungen und Anordnung dem Kontaktteil (35) entspricht. Wie bereits erwähnt besitzt das zweite Verbindungsteil (12b) ausserdem den Kontaktabschnitt (27), der derart angeordnet und geformt ist, dass er bei in das erste Verbindungsteil (8) eingeführtem zweitem Verbindungsteil (12b) mit den Kontaktteilen (29 und 30) so zusammenwirkt, dass zwischen beiden eine leitende Verbindung besteht.

### Bezugszeichenliste

- 1: Stimulationsimpuls-Generator
- 2: Detektorschaltung
- 3: Steuerlogik
- 4a: Ausgang
- 4b: Ausgangsanschluss
- 5a: Eingang
- 5b: Eingangsanschluss
- 6,7: Anschluss
- 8: Verbindungsteil
- 9: Gehäuse
- 10: bipolare Elektrode
- 11: Leitung
- 12a, 12: b zweites Verbindungsteil
- 13: Mittelleiter
- 14: äusserer Leiter
- 15: Spitze
- 16: Ring
- 17: unipolare Elektrode
- 18: Leitung
- 19: Leiter
- 20: Spitze
- 21, 21a, 21b: erster Pol
- 22: Umschalter
- 23, 23a: zweiter Pol
- 24: Sende/Empfangs-Spule
- 25: Telemetrieschaltkreis
- 26: dritter Pol
- 27: Kontaktabschnitt
- 28, 29, 30: Kontaktteil
- 31: Trägerteil
- 32: Bohrung
- 33, 34: Grundkörper
- 35, 36, 37: Kontaktteil

## Patentansprüche

1. Medizinisches Stimulationsgerät mit einer unipolaren und einer bipolaren Elektrode, wobei das Gerät wahlweise im unipolaren und bipolaren Betrieb arbeiten kann, und mindestens ein Pulsgenerator (1) zur Generierung von Stimulationsimpulsen und Verbindungsmittel (8) zum Anschliessen an das Gerät der unipolaren oder bipolaren Elektrode (10,17) aufweist, über welche die Stimulation an das Gewebe eines Lebewesens erfolgt, wobei der Pulsgenerator mit einem ersten Anschluss (6) und einem zweiten Anschluss (7) versehen ist, die ein Signalpotential beziehungsweise ein Bezugspotential führen, und wobei die Verbindungsmittel (8) mit einem ersten Pol (21) und einem zweiten Pol (23) versehen sind, wobei im Falle des unipolaren Betriebs der zweite Anschluss (7) mit Mitteln (9) zum Anlegen des Bezugspotentials an das Gewebe des Lebewesens verbunden ist und im Falle des bipolaren Betriebs der zweite Anschluss (7) mit dem zweiten Pol (23) verbunden ist, **dadurch gekennzeichnet** dass die Verbindungsmittel (8) mit einem dritten Pol (26) versehen sind der mit den Mitteln (9) leitend verbunden ist, wobei der dritte Pol (26) im Falle des bipolaren Betriebs bei dem die unipolare Elektrode (17) an das Gerät angeschlossen ist, leitend über ein Verbindungselement (27) der unipolaren Elektrode (17), den zweiten Anschluss (7) mit den Mitteln (9) verbindet.

2. Medizinisches Stimulationsgerät nach Anspruch 1, **dadurch gekennzeichnet** dass die Verbindungsmittel (8) als Steckverbindungsmittel (8) zum Anschliessen an das Gerät einer steckförmigen unipolaren oder bipolaren Elektrode (10,17) ausgebildet sind.

3. Medizinisches Stimulationsgerät nach Anspruch 2, **dadurch gekennzeichnet** dass die Steckverbindungsmittel (8) als koaxiale rotationssymmetrische Steckverbindungsmittel (8) ausgeführt ist, wobei die Pole (21,23,26) axial aufeinanderfolgend angeordnet sind und der zweite (23) und der dritte (26) Pol einander benachbart angeordnet sind.

4. Medizinisches Stimulationsgerät nach Anspruch 3, **dadurch gekennzeichnet** dass der erste (21), der zweite (23) und der dritte (26) Pol als ringförmige, jeweils mit Abstand zu einander angeordnete, Kontaktteile (28,29,30) ausgebildet sind.

5. Medizinisches Stimulationsgerät nach Anspruch 4, **dadurch gekennzeichnet** dass das Verbindungselement (27) der unipolaren Elektrode (17) durch einen an dieser Elektrode (17) angebrachten, ringförmigen Kontaktabschnitt (27) gebildet ist, wobei die axiale Länge des Kontaktabschnitts (27) nicht geringer als der Abstand zwischen den dem zweiten (23) und dem dritten (26) Pol entsprechenden Kontaktteilen (29,30) ist.

6. Medizinisches Stimulationsgerät nach einem der Ansprüche 1 - 5, **dadurch gekennzeichnet** dass das Gerät in den Körper des Lebewesens implantierbar ist und telemetrisch auf unipolaren oder bipolaren Betrieb umschaltbar ist.

7. Medizinisches Gerät nach einem der Ansprüche 1 - 6, **dadurch gekennzeichnet** dass die Mittel (9) zum anlegen des Bezugspotentials durch einen elektrisch leitenden Abschnitt der Aussenseite eines das Gerät umgebenden Gehäuses gebildet sind.

8. Medizinisches Gerät nach einem der Ansprüche 1 - 7, **dadurch gekennzeichnet** dass das Gerät als Herzschrittmacher ausgebildet ist, der Mittel (1) zum Stimulieren der Herztätigkeit des Lebewesens und Mittel (2) zum Detektieren von Herzschlägen aufweist, und an dem eine Elektrode (10,17) anschliessbar ist, die Stimulationsimpulse von den Mitteln 1 zum Stimulieren zu dem Herz des Lebewesens und/oder der elektrischen Aktivität des Herzens des Lebewesens entsprechende Signale von dem Herz des Lebewesens zu den Mitteln (2) zum Detektieren leitet.

## Claims

1. Medical stimulating apparatus with a unipolar and a bipolar electrode, wherein the apparatus can operate selectively in unipolar and bipolar mode, and exhibits at least one pulse generator (1) for generating stimulation pulses and connecting means (8) for connecting to the apparatus the unipolar or bipolar electrode (10, 17) via which the stimulation is effected to the tissue of a living organism, wherein the pulse generator is provided with a first connection (6) and a second connection (7), which carry a signal potential and a reference potential, respectively, and wherein the connecting means (8) are provided with a first pole (21) and a second pole (23), in which, in the case of the unipolar mode, the second connection (7) is connected to means (9) for the application of the reference potential to the tissue of the living organism and, in the case of the bipolar mode, the second connection (7) is connected to the second pole (23), characterised in that the connecting means (8) are provided with a third pole (26), which is conductively connected to the means (9), wherein in the case of the bipolar mode, in which the unipolar electrode (17) is connected to the apparatus, the third pole (26) conductively connects the second connection (7) to the means (9) via a connecting element (27) of the unipolar electrode (17).

2. Medical stimulating apparatus according to Claim 1, characterized in that the connecting means (8) are designed as plug connecting means (8) for the connection to the apparatus of a plug-type unipolar or bipolar electrode (10, 17).

3. Medical stimulating apparatus according to Claim 2, characterized in that the plug connecting means (8) are constructed as coaxial rotationally symmetric plug connecting means (8), in which the poles (21, 23, 26) are disposed axially in succession and the second pole (23) and the third pole (26) are disposed adjacent to one another.

4. Medical stimulating apparatus according to Claim 3, characterized in that the first pole (21), the second pole (23) and the third pole (26) are designed as annular contact parts (28, 29, 30) which are disposed in each instance at a spacing from one another.

5. Medical stimulating apparatus according to Claim 4, characterized in that the connecting element (27) of the unipolar electrode (17) is formed by an annular contact portion (27) which is fitted to this electrode (17), in which the axial length of the contact portion (27) is no smaller than the spacing between the contact parts (29, 30) corresponding to the second pole (29) and the third pole (26).

6. Medical stimulating apparatus according to one of Claims 1 to 5, characterized in that the apparatus is implantable into the body of the living organism and can be switched over by telemetry to unipolar or bipolar mode.

7. Medical apparatus according to one of Claims 1 to 6, characterized in that the means (9) for the application of the reference potential are formed by an electrically conductive portion of the external surface of a housing surrounding the apparatus.

8. Medical apparatus according to one of Claims 1 to 7, characterized in that the apparatus is designed as a cardiac pacemaker, which exhibits means (1) for the stimulation of the cardiac activity of the living organism and means (2) for the detection of heartbeats, and to which an electrode (10, 17) is connectable, which conducts stimulation pulses from the stimulating means (1) to the heart of the living organism and/or signals, corresponding to the electrical activity of the heart of the living organism from the heart of the living organism to the detecting means (2).

## Revendications

1. Appareil médical de stimulation, ayant une électrode unipolaire et une électrode bipolaire, l'appareil pouvant travailler au choix selon un fonctionnement unipolaire ou bipolaire et qui comporte au moins un générateur d'impulsions (1) servant à produire des impulsions de stimulation, et des moyens de liaison (8) pour le raccordement, à l'appareil, de l'électrode unipolaire ou bipolaire (10,17), au moyen de laquelle la stimulation est appliquée au tissu d'un être vivant, le générateur d'impulsions étant muni d'une première borne (6) et d'une seconde borne (7), qui appliquent un potentiel de signal ou un potentiel de référence, et les moyens de liaison (8) étant munis d'un premier pôle (21) et d'un second pôle (23), la seconde borne (7) étant reliée lors du fonctionnement unipolaire à des moyens (9) servant à appliquer le potentiel de référence au tissu de l'être vivant et, la seconde borne (7) étant reliée lors du fonctionnement bipolaire au second pôle (23), caractérisé par le fait que les moyens de liaison (8) sont munis d'un troisième pôle (26) qui est relié de façon conductrice aux moyens (9), et lors du fonctionnement bipolaire, lors duquel l'électrode unipolaire (17) est raccordée à l'appareil, le troisième pôle (26) est relié selon une liaison conductrice, par l'intermédiaire d'un élément de liaison (27) de l'électrode unipolaire (17), la seconde borne (7) aux moyens (9).

2. Appareil médical de stimulation suivant la revendication 1, caractérisé par le fait que les moyens de liaison (8) sont réalisés sous la forme de moyens de connexion à enfichage (8) pour le raccordement d'une électrode (10,17) unipolaire ou bipolaire en forme de fiche, à l'appareil.

3. Appareil médical de stimulation suivant la revendication 2, caractérisé par le fait que les moyens de connexion à enfichage (8) ont la forme de moyens de connexion à enfichage coaxiaux (8) à symétrie de révolution, les pôles (21,23,26) étant disposés axialement les uns à la suite des autres, tandis que le second pôle (23) et le troisième pôle (26) sont disposés l'un à côté de l'autre.

4. Appareil médical de stimulation suivant la revendication 3, caractérisé par le fait que le premier pôle (21), le second pôle (23) et le troisième pôle (26) ont la forme de pièces annulaires de contact (28,29,30), qui sont distantes les unes des autres.

5. Appareil médical de stimulation suivant la revendication 4, caractérisé par le fait que l'élément de liaison (27) de l'électrode unipolaire (17) est formée d'une section annulaire de contact (27), qui est disposée sur cette électrode (17), la longueur axiale de la section de contact (27) n'étant pas inférieure à la distance entre les pièces de contact (29,30), qui correspondent au second pôle (23) et au troisième pôle (26).

6. Appareil médical de stimulation suivant l'une des revendications 1 à 5, caractérisé par le fait que l'appareil peut être implanté dans le corps de l'être vivant et peut être commuté, par télémétrie, sur le fonctionnement unipolaire ou bipolaire.

7. Appareil médical de stimulation suivant l'une des revendications 1 à 6, caractérisé par le fait que les moyens (9) d'application du potentiel de référence sont constitués d'une section électriquement conductrice de la face extérieure d'un boîtier entourant l'appareil.

8. Appareil médical de stimulation suivant l'une des revendications 1 à 7, caractérisé par le fait que l'appareil a la forme d'un stimulateur cardiaque, qui comporte des moyens (1) pour stimuler l'activité cardiaque de l'être vivant et des moyens (2) pour détecter des battements cardiaques, et auquel peut être raccordée une électrode (10,17), qui envoie des impulsions de stimulation délivrées par les moyens (1) pour la stimulation du coeur de l'être vivant et/ou des signaux, qui correspondent à l'activité électrique du coeur de l'être vivant, fournis par le coeur de ce dernier aux moyens de détection (2).
